(19) Europäisches Patentamt
European Patent Office
Office européen des brevets

(11) Veröffentlichungsnummer: **0 176 913**
**A2**

(12) # EUROPÄISCHE PATENTANMELDUNG

(21) Anmeldenummer: 85112037.8

(22) Anmeldetag: 23.09.85

(51) Int. Cl.[4]: C 07 H 13/12
C 07 K 9/00, A 61 K 31/70
A 61 K 37/02

(30) Priorität: 05.10.84 DE 3436543

(43) Veröffentlichungstag der Anmeldung:
09.04.86 Patentblatt 86/15

(84) Benannte Vertragsstaaten:
AT BE CH DE FR GB IT LI LU NL SE

(71) Anmelder: BEHRINGWERKE Aktiengesellschaft
Postfach 1140
D-3550 Marburg 1(DE)

(72) Erfinder: Kolar, Cenek, Dr.
Deutschhausstrasse 20
D-3550 Marburg 1(DE)

(74) Vertreter: Meyer-Dulheuer, Karl-Hermann, Dr. et al,
HOECHST Aktiengesellschaft Zentrale Patentabteilung
Postfach 80 03 20
D-6230 Frankfurt/Main 80(DE)

(54) **Neue Glycourethanverbindungen, Verfahren zu ihrer Herstellung und ihre Verwendung.**

(57) Es werden Verbindungen der allgemeinen Formel I beschrieben

$$R^1-(CH_2)_n-CHR^2-CHR^3-NH-CO-O-R^4 \quad I$$

worin

$R^1$ ein Wasserstoffatom, eine Alkylgruppe mit 1 bis 5 C-Atomen, eine Carboxygruppe, eine Benzyloxycarbonylgruppe oder ein –CO-Träger,

$R^2$ ein Wasserstoffatom, eine Hydroxygruppe, eine tertiäre Alkyloxygruppe mit 4 bis 10 C-Atomen oder $-O-C(CH_3)_2-Ph(OCH_3)_m$ mit m = 0 bis 3,

$R^3$ ein Wasserstoffatom, eine Carboxygruppe, eine $C_1$- bis $C_4$-Alkyloxycarbonyl- oder Benzyloxycarbonylgruppe oder $-CO-A-NH-(CH_2)_a-CO-T$ mit

A ist ein Bindestrich oder ein in der Natur vorkommender L-Aminosäurerest, bei dem gegebenenfalls die reaktiven Gruppen, die nicht an der Peptidbindung teilnehmen, mit in der Peptidchemie üblichen Schutzgruppen geschützt sind, und

T eine Hydroxygruppe, eine O-Alkyl- oder O-Arylalkyl-Schutzgruppe, eine carboxygruppen-aktivierende Gruppe, ein Kephalinrest oder Träger bevorzugt ein Protein, Polypeptid, ein Aminogruppen-tragendes Gel oder ein Adsorbens und

a 1 bis 10,

$R^4$ ein mit in der Kohlenhydratchemie üblichen Schutzgruppen geschützter oder ungeschützter Glycosylrest, der sich von einer Glycopyranose, Glycofuranose oder einem Oligosaccharid ableitet und

n 0 bis 3 bedeuten,

sowie ein Verfahren zur ihrer Herstellung und ihre Verwendung, gebunden an einen Träger, als künstliche Antigene, Glycolipide, Inhibitoren oder Immunadsorbenzien.



Croydon Printing Company Ltd

BEHRINGWERKE AKTIENGESELLSCHAFT 84/B 021
Dr. Ha/Sd.

# Neue Glycourethanverbindungen, Verfahren zu ihrer Herstellung und ihre Verwendung

Die Erfindung betrifft neue Verbindungen der allgemeinen Formel I

$$R^1-(CH_2)_n-CHR^2-CHR^3-NH-CO-O-R^4 \qquad I$$

worin

$R^1$ ein Wasserstoffatom, eine Alkylgruppe mit 1 bis 5 C-Atomen, eine Carboxygruppe, eine Benzyloxycarbonylgruppe oder -CO-Träger,

$R^2$ ein Wasserstoffatom, eine Hydroxygruppe, eine tertiäre Alkyloxygruppe mit 4 bis 10 C-Atomen oder $-O-C(CH_3)_2-Ph(OCH_3)_m$ mit m = 0 bis 3,

$R^3$ ein Wasserstoffatom, eine Carboxygruppe, eine $C_1$- bis $C_4$-Alkyloxycarbonyl- oder Benzyloxycarbonylgruppe oder $-CO-A-NH-(CH_2)_a-CO-T$ mit

A ist ein Bindestrich oder ein in der Natur vorkommender L-Aminosäurerest, bei dem gegebenenfalls die reaktiven Gruppen, die nicht an der Peptidbindung teilnehmen, mit in der Peptidchemie üblichen Schutzgruppen geschützt sind, und

T eine Hydroxygruppe, eine O-Alkyl- oder O-Arylalkyl-Schutzgruppe, eine carboxygruppen-aktivierende Gruppe, ein Kephalinrest oder ein aminogruppen-tragendes Trägermolekül wie ein Protein, Polypeptid, Gel oder Adsorbens und

a 1 bis 10,

$R^4$ ein mit in der Kohlenhydratchemie üblichen Schutzgruppen geschützter oder ungeschützter Glycosylrest,

der sich von einer Glycopyranose, Glycofuranose oder einem Oligosaccharid ableitet und

n 0 bis 3

bedeutet, sowie ein Verfahren zur ihrer Herstellung und ihre Verwendung, gebunden an einen Träger, als künstliche Antigene, Glycolipide, Inhibitoren oder Immunadsorbenzien.

Für die Herstellung von synthetischen Kohlenhydrat-Antigenen oder Immunadsorbenzien werden chemische Verbindungen gebraucht, die mit geeigneten Trägermolekülen umgesetzt werden können.

Es ist bekannt, daß Kohlenhydrate, wenn sie über einen Spacer (Abstandhalter) an einen Träger gebunden werden, zu künstlichen Antigenen werden können.

Antigene Kohlenhydrat-Determinanten sind Oligosaccharid-Strukturen, die als Bestandteil der Glycoproteine oder Glycolipide eine biologische Funktion besitzen. Die unter $R^4$ bezeichneten Kohlenhydratreste können entweder aus biologischem Material isoliert oder chemisch hergestellt werden. Solche Monosaccharid- oder Oligosaccharid-Reste sollten über das reduktive Ende gekoppelt werden, damit ihre biologische Spezifität erhalten bleibt. Es ist zweckmäßig, solche Kohlenhydrate, die selbst nicht immunogen sind, mittels einer 5 bis 12-atomigen Kette (Spacer) an Trägermoleküle zu binden, um zu immunogenen Antigenen oder aktiven Immunadsorbenzien zu gelangen.

Überraschenderweise hat es sich bei einer beabsichtigten Herstellung von Glycopeptiden gezeigt (siehe Schema 1), daß statt des gewünschten O-glycosidisch gebundenen Glycopeptids durch eine Umlagerungsreaktion unter Beteiligung der DDZ-Schutzgruppe eine neue Glycosylurethan-Verbindung entstanden war.

Schema 1

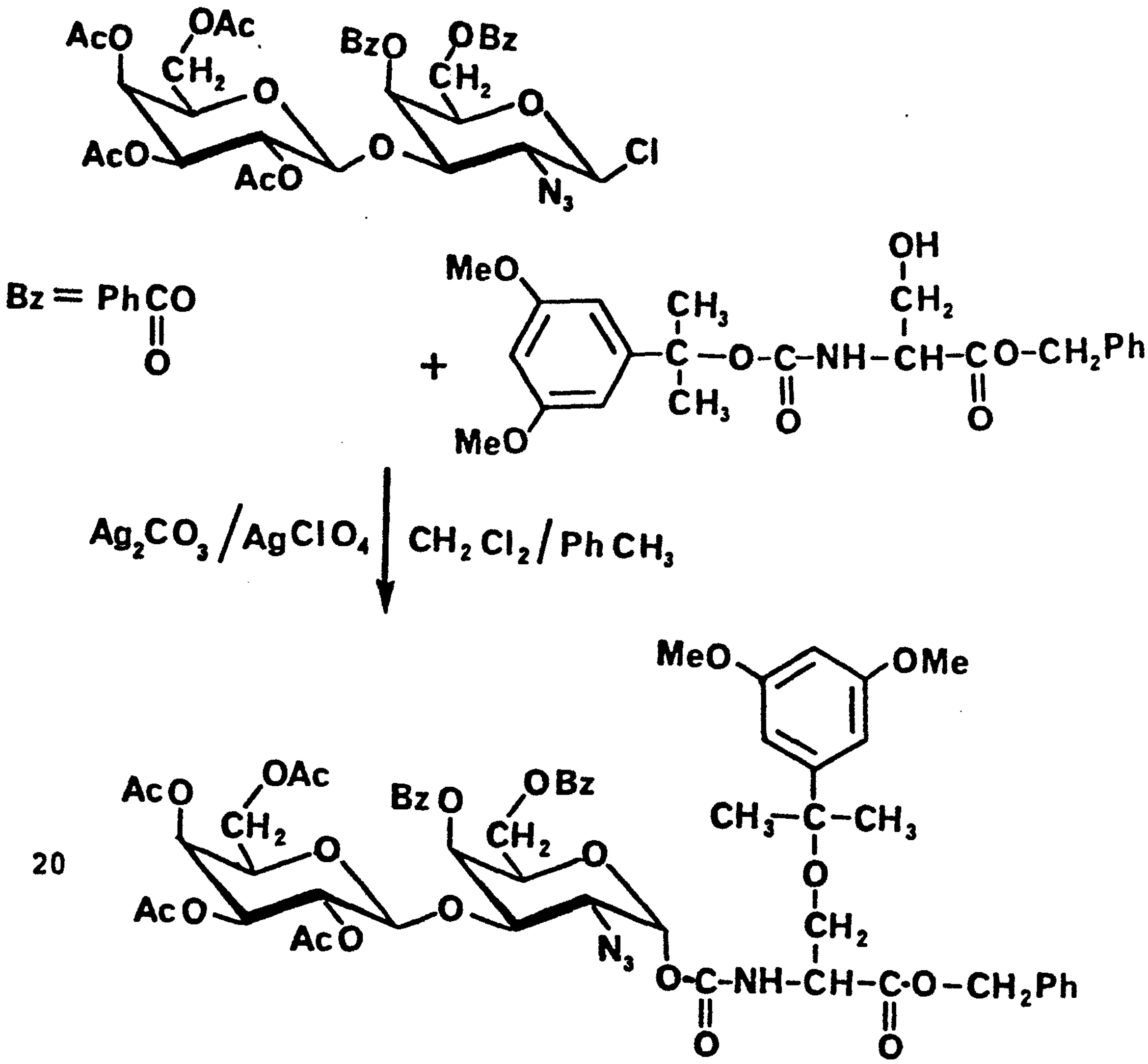


Hiermit eröffnete sich ein Weg zur Herstellung von neuen Glycokonjugaten sowie die Möglichkeit, Oligosaccharid-Segmente mittels dieser neuen Spacer-Verknüpfungsmöglichkeit kovalent an Träger zu binden.

Durch diese Umlagerungsreaktion wurde die Hydroxygruppe des Serin-Segmentes mittels alpha,alpha-Dimethyl-3,5-dimethoxybenzylgruppe verethert.
Da diese Gruppe basenstabil ist, jedoch in sauerem Medium leicht abspaltbar ist, kann sie als neue Schutzgruppe für alkoholische Hydroxygruppen betrachtet werden.

Die vorliegende Erfindung hatte sich zur Aufgabe gestellt, neue Kopplungsverbindungen und Kopplungsmethoden zu entwickeln, um biologisch aktive Kohlenhydrat-Haptene kovalent an Trägermoleküle zu binden.

Gelöst wird diese Aufgabe durch die Herstellung von Verbindungen der allgemeinen Formel I und ihre Bindung an Träger.

Bevorzugt sind Verbindungen der Formel I worin

$R^1$ ein Wasserstoffatom, eine Alkylgruppe mit 1 bis 5 C-Atomen, eine Carboxygruppe, eine Benzyloxycarbonylgruppe oder -CO-Träger; wobei der Träger Albumin, Polylysin oder ein aminogruppen-tragendes Gel ist,

$R^2$ ein Wasserstoffatom, eine Hydroxygruppe oder eine tert.-Butyloxy- oder alpha,alpha-Dimethyl-3,5-dimethoxy-benzylgruppe,

$R^3$ ein Wasserstoffatom, eine Carboxygruppe, eine Methyloxycarbonyl- oder Benzyloxycarbonylgruppe oder $CO-A-NH-(CH_2)_5-CO-T$ mit

A ist ein Bindestrich oder ein L-Aminosäurerest von Val, Ser, Glu, Glu(gamma-t.-But), Leu oder Ile und

T eine Hydroxygruppe, eine Methoxy- oder Benzyloxygruppe, eine carboxygruppen-aktivierende Gruppe wie die O-(N-succinimid)- oder Nitrophenyloxygruppe, ein Kephalinrest oder Albumin, Polylysin oder ein aminogruppen-tragendes Gel als Träger,

$R^4$ ein Glycosylrest der Formel II

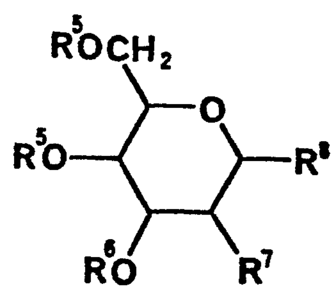

II

in der

$R^5$ ein Wasserstoffatom, eine Acylschutzgruppe, wie Acetyl-, Benzoylgruppe oder eine Benzylgruppe,
$R^6$ ein Wasserstoffatom, eine Acylschutzgruppe, eine Benzylgruppe, ein 2,3,4,6-Tetra-O-acetyl-ß-D-galactopyranosyl- oder ß-D-Galactopyranosylrest,
$R^7$ ein Wasserstoffatom, eine Hydroxy-, Azido-, Amino-, Acetamido- oder Benzyloxy- oder Trichloracetyloxygruppe und
$R^8$ ein Bindestrich ist, und n = 0 bis 3 bedeutet.

Das erfindungsgemäße Verfahren zur Herstellung einer der neuen Verbindungen der Formel I ist dadurch gekennzeichnet, daß man

a) eine Verbindung der allgemeinen Formel III

$R^1-(CH_2)_n-CHR^2-CHR^3-NH-CO-O-R^9$ III

in der

$R^1$ ein Wasserstoffatom, eine Alkylgruppe mit 1 bis 5 C-Atomen oder eine Benzyloxycarbonylgruppe,
$R^2$ ein Wasserstoffatom oder eine Hydroxygruppe,
$R^3$ ein Wasserstoffatom oder eine $C_1$ bis $C_4$-Alkyloxycarbonyl- oder Benzyloxycarbonylgruppe und
$R^9$ eine tertiäre Alkyloxygruppe mit 4 bis 10 C-Atomen oder $-O-C(CH_3)_2-Ph(OCH_3)_m$ mit m = 0 bis 3 bedeuten,

mit einer Verbindung der allgemeinen Formel II, in der

$R^5$ eine Acylschutzgruppe oder eine Benzylgruppe,
$R^6$ eine Acylschutzgruppe, eine Benzylgruppe oder ein 2,3,4,6-Tetra-O-acetyl-ß-D-galactopyranosylrest,

$R^7$ ein Wasserstoffatom, eine Hydroxy-, Azido-, Benzyloxy- oder Trichloracetyloxygruppe und

$R^8$ Chlor oder Brom bedeuten,

in einem organischen Lösungsmittel, wie DMF, Dioxan, THF, Essigsäureethylester, Toluol, Chloroform oder Dichlormethan oder deren zwei- oder drei-Komponenten-Mischungen in Gegenwart von Silber-I- oder Quecksilber-II-Salzen und eines Säurefängers wie Polyvinylpyridin oder Molekularsiebes und gegebenenfalls eines Trockenmittels wie Calciumsulfat oder Magnesiumsulfat bei -50°C bis +80°C, bevorzugt bei -30° bis +50°C, zu einer Verbindung der allgemeinen Formel I umsetzt, worin

$R^1$ unverändert,

$R^2$ ein Wasserstoff, eine Hydroxygruppe, eine tertiäre Alkyloxygruppe mit 4 bis 10 C-Atomen oder $-O-C(CH_3)_2-Ph(OCH_3)_m$ mit m = 0 bis 3,

$R^3$ unverändert und

$R^4$ ein Glycosylrest der Formel II, in dem die Reste $R^5$, $R^6$ und $R^7$ unverändert bleiben und $R^8$ ein Bindestrich ist, und

n = 0 bis 3 sind;

b) in einem Produkt von Stufe a), das als Benzylester vorliegt, die Benzyl-Schutzgruppe in Gegenwart eines Hydrierungskatalysators wie Palladium/Kohle und eines organischen Lösungsmittels, wie Essigsäureethylester oder Dioxan bevorzugt bei Raumtemperatur hydrogenolytisch abspaltet, wobei die Azidogruppe des Kohlenhydratsegmentes unverändert bleibt und gegebenenfalls das entstandene Produkt, das eine freie Carboxylgruppe aufweist, zu einem in der Peptid-Chemie üblichen Aktivester-Derivat wie einem Succinimidester, p-Nitro-phenylester oder Anhydrid umsetzt;

c) ein Produkt von Stufe b) nach einem in der Peptidchemie üblichen Kondensationsverfahren mit einer Aminoverbindung der allgemeinen Formel IV

$$H\text{-}A\text{-}NH\text{-}(CH_2)_a\text{-}CO\text{-}T \qquad IV$$

umsetzt, worin

T eine O-Alkyl- oder O-Arylalkylschutzgruppe,

A ein Bindestrich oder L-Aminosäurerest, dessen reaktive Gruppen, die nicht an der Kondensationsreaktion teilnehmen, mittels Schutzgruppen geschützt sind, und

a 1 bis 10 bedeuten;

d) in einem Produkt von Stufe a) oder d), das als tert. Alkyl- oder Arylalkylether vorliegt, in an sich bekannter Weise die tert. Butyl- oder DDBn-Schutzgruppe in Gegenwart einer Säure wie Trifluoressigsäure oder Essigsäure/HCl und gegebenenfalls eines Lösungsmittels wie Dioxan, Methanol oder Essigsäureethylester abspaltet, wobei das Produkt als Alkoholderivat vorliegt;

e) in einem Produkt von Stufe d) die restlichen Schutzgruppen nach in der Kohlenhydratchemie oder Peptidchemie üblichen Entblockierungsverfahren abspaltet und eine Verbindung, die eine Carboxygruppe am Spacer trägt, nach üblichen Kondensationsverfahren mit einem aminierten Träger umsetzt.

Die Glycourethanderivate der allgemeinen Formel I werden nach einem Verfahren hergestellt, wie es üblicherweise in der Peptidchemie, zum Beispiel in Houben-Weyl, Methoden der organischen Chemie, Band 15, angewandt wird.

Die Aktivierung der Carboxygruppe kann erreicht werden zum Beispiel durch Umwandlung der Carboxygruppe in ein Säurehalogenid, ein Azid, Ahydrid, Imidazolid oder einen

aktivierten Ester wie den N-Hydroxysuccinimidester oder den p-Nitrophenylester.

Die Aminogruppe kann aktiviert werden durch Umwandlung in ein Phosphitamid oder nach dem Phosphorazo-Verfahren.

Die üblichen Verfahren für die oben angegebenen Kondensationsreaktionen sind: Das Carbodiimidverfahren, das Azidverfahren, das gemischte Anhydridverfahren und das Verfahren der aktivierten Ester, wie sie beschrieben sind in "The Peptides", Band 1, 1965 (Academic Press).

Die reaktionsfähigen Gruppen, die nicht an der Kondensationsreaktion teilnehmen sollen, werden durch Schutzgruppen geschützt, die leicht wieder entfernt werden können, zum Beispiel durch Hydrolyse oder Reduktion. Wegen einer Beschreibung der Schutzgruppen wird auf die entsprechende Literatur verwiesen (Houben-Weyl, Methoden der org. Chemie, Band 15).

Es ist zu empfehlen, die gamma-Carboxygruppe von Glutaminsäure zu schützen. Übliche Schutzgruppen in diesem Zusammenhang sind eine tert.-Butyl-, eine Benzyl- oder eine Methylgruppe.

Die Schutzgruppen können nach üblichen Verfahren abgespalten werden, je nach Art der jeweiligen Gruppe zum Beispiel mit Trifluoressisäure oder durch milde Reduktion, zum Beispiel mit Wasserstoff und einem Katalysator wie Palladium oder mit HBr in Eisessig.

Die Schutzgruppen am Kohlenhydratsegment können je nach Art der Schutzgruppe nach üblichen Verfahren abgespalten werden (Angew. Chem. (1982), 94, 184; Carbohydr. Res. (1983), 116, C9-C12). Die Azidogruppe kann durch milde Reduktion z.B. mit Wasserstoff und einem Katalysator,

wie Palladium oder mit Natriumborhydrid in Gegenwart von Nickel (II)-chlorid, zu einer Aminogruppe umgesetzt werden, die durch Acetylierung z.B. mit Acetanhydrid in Methanol in die Acetylaminogruppe überführt wird. Die O-Acylschutzgruppen werden vorteilhaft im basischen Milieu z.B. mit Natriummethylat in Methanol oder Natriumhydroxyd oder Natriumcarbonat in Methanol abgespalten. Die Benzylgruppen können hydrogenolytisch in Gegenwart von Palladium/Kohle und Eisessig abgespalten werden.

Die erfindungsgemäßen Glycourethane der Formel I werden nach üblichen Verfahren an der Carboxygruppe des Spacers in Aktivesterderivate überführt, wie den N-Hydroxysuccinimidester oder den p-Nitrophenylester und an Träger, der eine oder mehrere Aminogruppen besitzt, gekoppelt. Es ist hierbei besonders zu empfehlen, die reaktionsfähigen Gruppen einer Verbindung der Formel I, die nicht an der Kondensationsreaktion teilnehmen, durch Schutzgruppen zu schützen. Die Schutzgruppen können wieder z.B. durch Hydrolyse oder Photolyse entfernt werden.

Träger sind z.B. Proteine, bevorzugt Human- oder Rinderserum-Albumin, Glycoproteine, Polymere wie Polylysin oder Poly(glycyllysin), aktivierte Gele, die Amino-, Glycidyl-, 2-Aminoethylamino- oder Aktivester-Gruppe tragen, Bromcyan-aktivierte Polysaccharide oder Polysaccharid-Gele oder Lipide, bevorzugt Kephaline oder aminierte Phospholipide.

Die erfindungsgemäßen Träger-gebundenen Glycourethan-Derivate sind künstliche Antigene, Glycolipide oder Immunadsorbentien.

Die folgenden Beispiele erläutern die Erfindung.

Bezüglich der verschiedenen in der Beschreibung, den Beispielen und den Patentansprüchen verwendeten Abkürzungen ist folgendes zu bemerken:

I. Wenn bei Aminosäureresten keine optische Konfiguration angegeben ist, ist die L-Form gemeint;

folgende übliche Abkürzungen für Aminosäurereste werden verwendet:

| | | |
|---|---|---|
| Val | = | L-valyl |
| Ser | = | L-seryl |
| Glu | = | L-glutamyl |
| Glu (gamma-t.But) | = | L-glutamyl (gamma-tert.-butyl) |
| Leu | = | L-leucyl |
| Ile | = | L-isoleucyl |

II. Folgende Abkürzungen werden verwendet, um die Schutzgruppen oder aktivierende Gruppen zu bezeichnen:

| | | |
|---|---|---|
| DDZ | = | alpha,alpha-Dimethyl-3,5-dimethoxy-benzyloxycarbonyl |
| Z | = | Benzyloxycarbonyl |
| BOC | = | tert.-Butyloxycarbonyl |
| t.-But | = | tert.-Butyl |
| Me | = | Methyl |
| pNP | = | p-Nitrophenyl |
| Bn | = | Benzyl |
| Bz | = | Benzoyl |
| Ph | = | Phenyl |
| DDBn | = | alpha,alpha-Dimethyl-3,5-dimethoxy-benzyl |
| Gal | = | D-Galactopyranosyl |
| GalNAc | = | N-Acetyl-D-galactopyranosyl |

Experimenteller Teil

Die in den folgenden Beispielen angeführten Verbindungen wurden wie folgt charakterisiert:

IR-spektroskopisch

Vor allem auf die Anwesenheit der funktionellen Gruppen wie Hydroxy-, Amino-, Amido-, Carbonyl-, Azido-, Aryl- und Alkylgruppen.

NMR-spektroskopisch

mittels $^{1}H$-, $^{13}C$- und $^{2}D$-NMR-Spektren

Massenspektrokopisch

Ermittlung der Molmasse, vor allem bei den Umlagerungsprodukten

mittels Elementaranalyse

Dünnschichtchromatographisch oder mittels HPLC-Technik

mittels Aminosäurenanalyse

Beispiel 1:

Herstellung von Glycosylurethanen

Für die Herstellung der Glycosylurethane wurden folgende Glycosylhalogenide eingesetzt:

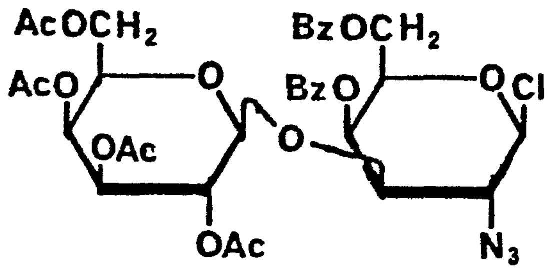

Bz=PhCO

Verbindung 1

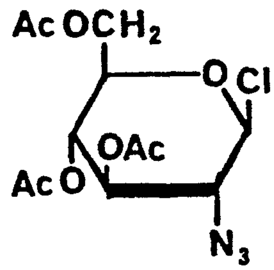

Verbindung 2

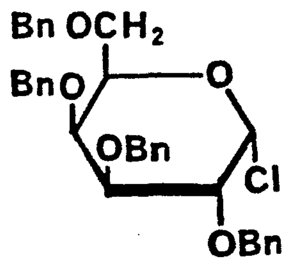

Verbindung 3

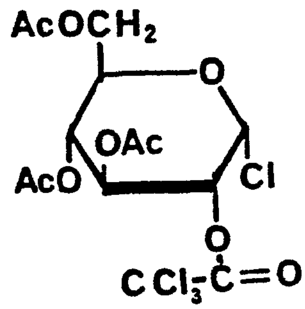

Verbindung 4

Herstellung von Verbindung 5

N-((3-O-(2,3,4,6-Tetra-O-acetyl-ß-D-galactopyranosyl)-2-azido-4,6-di-O-benzoyl-2-desoxy-alpha-D-galactopyranosyl)-oxycarbonyl)-O-(alpha,alpha-dimethyl-3,5-dimethoxy-benzyl)-L-serin-benzylester

10 mmol Verbindung 1 und 12 mmol DDZ-Ser-O-Bn wurden in trockenem Dichlormethan/Toluol (1:1; 100 ml) gelöst und mit einem Molekularsieb 4 A (10 g), Calciumsulfat (8 g; geglüht) und Silbercarbonat (11 g) 30 min bei 20°C gerührt. Danach fügte man Silberperchlorat (1,1 g) hinzu. Nach 20 h Rühren unter Lichtschutz war die Reaktion beendet. Es wurde mit trockenem Triethylamin neutralisiert, abfiltriert und in vacuo eingeengt. Das Produkt wurde säulenchromatographisch an Kieselgel 60 (70-230 mesh) gereinigt.

Ausbeute: 70% der Theorie bezogen auf die Glycosyl-Komponente.
(alpha)$_D^{20}$ = + 50,8° (c = 1 in Chloroform)

Herstellung von Verbindung 6

N-((3-O-(2,3,4,6-Tetra-O-acetyl-ß-D-galactopyranosyl)-2-azido-4,6-di-O-benzoyl-2-desoxy-alpha-D-galactopyranosyl)-oxycarbonyl)-O-(tert.-butyl)-L-serin-benzylester

Die Verbindung 6 wurde ausgehend von Verbindung 1 und BOC-Ser-O-Bn nach dem oben beschriebenen Verfahren hergestellt.

Herstellung von Verbindung 7

N-((3,4,6-Tri-O-acetyl-2-azido-2-desoxy-alpha-D-glucopyranosyl)-oxycarbonyl)-O-(alpha,alpha-dimethyl-3,5-dimethoxybenzyl)-aminoethanol

Verbindung 7 wurde ausgehend von Verbindung 2 und N-DDZ-Aminoethanol nach dem oben beschriebenen Verfahren hergestellt.
Ausbeute: 68 %, (alpha)$_D^{20}$ = + 65,3° (c = 1 in Chloroform)

Herstellung von Verbindung 8

N-(2,3,4,8-Tetra-O-benzyl-ß-D-galactopyranosyl-oxycarbonyl)-n-pentylamin

Die Verbindung 8 wurde ausgehend von Verbindung 3 und N-DDZ-pentylamin nach dem oben beschriebenen Verfahren hergestellt.

Herstellung von Verbindung 9

N-(3,4,6-Tri-O-acetyl-2-trichloracetyl-ß-D-glucopyrano-

syl-oxycarbonyl)-O-(alpha,alpha-dimethyl-3,5-dimethoxy-benzyl)-aminoethanol

Die Verbindung 9 wurde ausgehend von Verbindung 4 und N-DDZ-Aminoethanol hergestellt.

Herstellung von Verbindung 10

N-(3,4,6-Tri-O-acetyl-2-azido-2-desoxy-alpha-D-glucopyranosyl-oxycarbonyl)-6-amino-hexansäurebenzylester

Die Verbindung 10 wurde ausgehend von Verbindung 2 und N-DDZ-6-Aminohexansäurebenzylester hergestellt.
Ausbeute: 85 %, $(alpha)_D^{20}$ = + 61° (c = 1 in Chloroform)

Bespiel 2:

Selektive hydrogenolytische Abspaltung der Benzylgruppe von den Benzylester-Verbindungen 5, 6 und 10

Allgemeine Arbeitsvorschrift

3 mmol Benzylester-Verbindung wurde in 70 ml trockenem Essigsäureethylester gelöst und in Gegenwart von 3,5 g 10%-igem Palladium auf Kohle 2 h bei Raumtemperatur hydriert. Dann wurde abfiltriert, nachgewaschen und in vacuo zum Sirup eingeengt.
Das resultierende Produkt wurde säulenchromatographisch an Kieselgel gereinigt. Ausbeute: 92 %

Herstellung von Verbindung 11

N-((3-O-(2,3,4,6-Tetra-O-acetyl-ß-D-galactopyranosyl)-2-azido-4,6-di-O-benzoyl-2-desoxy-alpha-D-galactopyranosyl)-oxycarbonyl)-O-(alpha,alpha-dimethyl-3,5-dimethoxy-benzyl)-L-serin.
$(alpha)_D^{20}$ = + 50,6° (c = 1 in Chloroform)

Herstellung von Verbindung 12

N-((3-O-(2,3,4,6-Tetra-O-acetyl-β-D-galactopyranosyl)-2-azido-4,6-di-O-benzoyl-2-desoxy-alpha-D-galactopyranosyl)-oxycarbonyl)-O-(tert.-butyl)-L-serin.
$(\alpha)_D^{20}$ = + 51,6° (c = 1 in Chloroform)

Beispiel 3:

Entblockierung von Verbindung 10

Herstellung von Verbindung 13

N-(2-Acetamido-2-desoxy-alpha-D-glucopyranosyl-oxycarbonyl)-6-aminohexansäure

5 mmol Verbindung 10 wurden in 50 ml trockenem Eisessig gelöst und in Gegenwart von 500 mg 10%-igem Palladium auf Kohle bei Raumtemperatur 20 h hydriert. Anschließend wurden 10 ml Acetanhydrid zugegeben und weitere 5 h gerührt. Nach Abfiltrieren des Katalysators wurde die Lösung in vacuo eingeengt und der resultierende Sirup mehrmals mit Toluol nachdestilliert. Das Produkt wurde in wäßrigem Methanol gelöst und der pH-Wert der Reaktionsmischung mit Sodalösung auf pH 9,5 eingestellt. Nach 24 h Rühren wurde mit aktiviertem Ionenaustauscher neutralisiert, abfiltriert und in vacuo zum Sirup eingeengt. Das Produkt wurde noch säulenchromatographisch (Sephadex LH-20) gereinigt.
Ausbeute: 74 %

Beispiel 4:

Kondensation der Verbindungen 11 oder 12 mit einer Amino-Komponente

Die Verbindungen 11 oder 12 wurden zunächst nach der folgenden allgemeinen Arbeitsvorschrift in den Aktivester überführt:

a) Herstellung des "Aktivesters"
Ein Carbonsäurederivat (3 mmol), das nur eine freie Carboxygruppe aufweist, und bei dem die übrigen reaktiven Gruppen geschützt sind, wurde in 50 ml trockenem Acetonitril gelöst. N-Hydroxysuccinimid (3 mmol) und Dicyclohexylcarbodiimid (3 mmol) wurden unter Rühren zugegeben. Nach 24 h wurde der Reaktionsansatz bei 0°C abfiltriert und in vacuo eingeengt. Anschließend wurde der Sirup mit Toluol (25 ml) versetzt und in vacuo eingeengt. Der resultierende Sirup wurde ohne weitere Reinigung im nächsten Reaktionsschritt verwendet.
Häufig verwendetes Laufmittelsystem für Dünnschichtchromatographie: Chloroform/Methanol 9:1, 7:1, 3:1 und 1:1; Chloroform/Essigsäureethylester 1:1.

b) Herstellung der Peptidbindung
Ein Aktivester der Verbindung 11 oder 12 wurde mit einer Amino-Verbindung der Formel IV nach folgender Arbeitsvorschrift umgesetzt:

<u>Amino-Verbindungen</u>

| | |
|---|---|
| $H_2N-(CH_2)_5-COO$-Benzyl | Verbindung 14 |
| $H_2N-(CH_2)_5-COO$-Methyl | Verbindung 15 |
| H-Val-NH-$(CH_2)_5$-COO-Methyl | Verbindung 16 |
| H-Ser-NH-$(CH_2)_5$-COO-Methyl | Verbindung 17 |
| H-Leu-NH-$(CH_2)_5$-COO-Methyl | Verbindung 18 |
| H-Ile-NH-$(CH_2)_5$-COO-Methyl | Verbindung 19 |
| H-Glu(gamma-tert.-Butyl)-NH-$(CH_2)_5$-COO-Benzyl | Verbindung 20 |

c) Vorschrift zur Herstellung der Peptidbindung

Eine Amino-Verbindung (3 mmol) wurde in trockenem Chloroform (25 ml) gelöst und unter Rühren mit ca. 3 mmol 4-(N,N-Dimethyl-amino)-pyridin auf pH 9 eingestellt. Nach 10 min wurde der in 25 ml trockenem Chloroform gelöste "Aktivester" von der Stufe a) zugegeben. Nach 24 h wurde der Reaktionsansatz einmal mit 5%-iger (w:v) Citronensäure ausgewaschen, mit Natriumsulfat getrocknet und in vacuo eingeengt. Der resultierende Sirup wurde säulenchromatographisch an Kieselgel gereinigt.

Ausbeute: 70 bis 85 % der Theorie bezogen auf Verbindung 11 oder 12.

Häufig verwendete Laufmittelsysteme für Chromatographie: Chloroform/Essigsäureethylester 1:1; Chloroform/Aceton 7:1, 4:1 und 1:1; Chloroform/Methanol 9:1 und 5:1.

Nach diesen Vorschriften wurden folgende Verbindungen hergestellt:

Verbindung 21 (aus Verbindung 12 (Aktivester) und Verbindung 15)

N-((3-O-(2,3,4,6-Tetra-O-acetyl-ß-D-galactopyranosyl)-2-azido-4,6-di-O-benzoyl-2-desoxy-alpha-D-galactopyranosyl)-oxycarbonyl)-O-(tert.-butyl)-L-seryl-5-methoxycarbonyl-pentylamid.

$(alpha)_D^{20}$ = + 60,8° (c = 1 in Chloroform)

Verbindung 22 (aus Verbindung 11 (Aktivester) und Verbindung 14)

N-((3-O-(2,3,4,6-Tetra-O-acetyl-ß-D-galactopyranosyl)-2-azido-4,6-di-O-benzoyl-2-desoxy-alpha-D-galactopyranosyl)-oxycarbonyl)-O-(alpha,alpha-dimethyl-3,5-dimethoxybenzyl)-L-seryl-5-benzyloxycarbonyl-pentylamid.

(alpha)$_D^{20}$ = + 54,2° (c = 1 in Chloroform)

Verbindung 23 (aus Verbindung 11 (Aktivester) und Verbindung 16)

N-((3-O-(2,3,4,6-Tetra-O-acetyl-ß-D-galactopyranosyl)-2-azido-4,6-di-O-benzoyl-2-desoxy-alpha-D-galactopyranosyl)-oxycarbonyl)-O-(alpha,alpha-dimethyl-3,5-dimethoxy-benzyl)-L-seryl-L-valyl-5-methoxycarbonyl-pentylamid
(alpha)$_D^{20}$ = + 41,8° (c = 1 in Chloroform)

Verbindung 24 (aus Verbindung 11 (Aktivester) und Verbindung 17)

N-((3-O-(2,3,4,6-Tetra-O-acetyl-ß-D-galactopyranosyl)-2-azido-4,6-di-O-benzoyl-2-desoxy-alpha-D-galactopyranosyl)-oxycarbonyl)-O-(alpha,alpha-dimethyl-3,5-dimethoxy-benzyl)-L-seryl-L-seryl-5-methoxycarbonyl-pentylamid
(alpha)$_D^{20}$ = + 41,2° (c = 1 in Chloroform)

Verbindung 25 (aus Verbindung 11 (Aktivester) und Verbindung 18)

N-((3-O-(2,3,4,6-Tetra-O-acetyl-ß-D-galactopyranosyl)-2-azido-4,6-di-O-benzoyl-2-desoxy-alpha-D-galactopyranosyl)-oxycarbonyl)-O-(alpha,alpha-dimethyl-3,5-dimethoxy-benzyl)-L-seryl-L-leucyl-5-methoxycarbonyl-pentylamid

Verbindung 26 (aus Verbindung 11 (Aktivester) und Verbindung 19)

N-((3-O-(2,3,4,6-Tetra-O-acetyl-ß-D-galactopyranosyl)-2-azido-4,6-di-O-benzoyl-2-desoxy-alpha-D-galactopyranosyl)-oxycarbonyl)-O-(alpha,alpha-dimethyl-3,5-dimethoxy-benzyl)-L-seryl-L-isoleucyl-5-methoxycarbonyl-pentylamid

Verbindung 27 (aus Verbindung 11 (Aktivester) und Verbindung 20)

N-((3-O-(2,3,4,6-Tetra-O-acetyl-ß-D-galactopyranosyl)-2-azido-4,6-di-O-benzoyl-2-desoxy-alpha-D-galactopyranosyl)-oxycarbonyl)-O-(alpha,alpha-dimethyl-3,5-dimethoxy-benzyl)-L-seryl-L-glutamyl(gamma-tert.-butyl)-5-benzyloxycarbonyl-pentylamid
$(alpha)_D^{20}$ = + 23,5° (c = 1 in Chloroform)

Beispiel 5:

Entblockierungsreaktionen

Die Entblockierungsreaktionen wurden nach folgenden allgemeinen Arbeitsvorschriften (AAV) durchgeführt:

AAV 1: Selektive hydrolytische Abspaltung der alpha, alpha-Dimethyl-3,5-dimethoxy-benzyl(DDBn)-Schutzgruppe

Die DDBn-Schutzgruppen-tragende Verbindung (3 mmol) wurde in 50 ml 5 vol.-%igem Trifluoressigsäure-haltigem Dichlormethan bei Raumtemperatur gelöst. Nach 30 min Rühren wurde mit N-Methylmorpholin neutralisiert. Die Mischung wurde einmal mit Eiswasser, dann mit verdünnter Salzsäure gewaschen. Die organische Phase wurde mit Natriumsulfat getrocknet und in vacuo zum Sirup eingeengt. Das Produkt liegt als Hydrochlorid vor.
Ausbeute: 96 bis 99 %

Dünnschichtchromatographische Kontrolle wurde mit folgenden Laufmittelsystemen durchgeführt:
Chloroform/Essigsäureethylester 1:1; Chloroform/Aceton 4:1 und 1:1; Chloroform/Methanol 4:1 und 1:1.

<u>AAV 2</u>: Selektive hydrolytische Abspaltung der tert.-Butyl(t.-But.)-Schutzgruppe

Das t.-But-Schutzgruppen-tragende Produkt (2,6 mmol) wurde in 10 ml 1,2 normalem Salsäure-haltigen trockenen Eisessig bei Raumtemperatur gelöst. Nach 20 min Rühren wurde der Reaktionsansatz mit 20 ml Ether versetzt und in vacuo eingeengt. Der resultierende Sirup wurde mehrmals mit Toluol versetzt und eingeengt, bis der Geruch von Essigsäure nicht mehr erkennbar war. Das resultierende einheitliche Produkt wurde säulenchromatographisch an Kieselgel gereinigt.
Ausbeute: 83 bis 85 %

Häufig verwendete Laufmittelsysteme für Chromatographie: Chloroform/Aceton 9:1 und 4:1; Chloroform/Methanol 9:1 und 5:1.

<u>AAV 3</u>: Selektive hydrolytische Reduktion der 2-Azidogruppe zur 2-Aminogruppe am Galactose-Baustein und anschließende Acetylierung zu Acetamidogruppen

a) Reduktion der Azidogruppe zur Aminogruppe
In einer Suspension aus 500 mg 10%-igem Palladium auf Kohle und 100 ml trockenem Methanol wurde ca. 30 min Wasserstoff eingeleitet. Mit einem Gemisch aus Methanol und wäßriger konzentrierter Soda-Lösung (50:1) wurde der pH-Wert der Suspension auf pH 7 eingestellt. Die Azid-Verbindung (5 mmol), gelöst in wenig Methanol, wurde zugegeben und anschließend 3 h unter Lichtschutz hydriert. Im Verlauf der Hydrierung wurde der pH-Wert kontrolliert und, falls erforderlich, auf pH 7 eingestellt. Dann wurde filtriert, sorgfältig mit Methanol/Ether nachgewaschen und in vacuo zum Sirup eingeeengt.

b) Acetylierung

Das Produkt von Stufe a) wird in trockenem Methanol gelöst und mit Acetanhydrid (50 mmol) versetzt. Nach 24 h Rühren wurde der Reaktionsansatz in vacuo eingeengt und der resultierende Sirup mehrmals mit Toluol versetzt und in vacuo eingeengt. Das einheitliche Produkt wurde säulenchromatographisch an Kieselgel gereinigt.

Ausbeute: 72 bis 75 %

Häufig verwendete Laufmittelsysteme zur Chromatographie: Chloroform/Aceton 9:1, 5:1 und 2:1, Chloroform/Methanol 9:1 und 6:1.

AAV 4: Selektive hydrolytische Abspaltung der Benzyl-Schutzgruppe am Kohlenhydrat

Die Benzylgruppe-tragende Verbindung (3 mmol) wurde in 70 ml trockener Essigsäure gelöst und in Gegenwart von 3,5 g 10%-igem Palladium auf Kohle 2 h hydriert. Dann wurde filtriert, nachgewaschen und in vacuo zum Sirup eingeengt. Das resultierende Produkt wurde säulenchromatographisch gereinigt.

Ausbeute: 89 bis 94 %

Laufmittel für Chromatographie: Chloroform/Aceton 4:1 und 1:1; Chloroform/Methanol 5:1 und 1:1.

AAV 5: Abspaltung der O-Acyl-Schutzgruppen am Kohlenhydrat-Baustein

Die Acyl-Verbindung (2 mmol) wurde in 30 ml Methanol gelöst. Zu der Lösung wurde tropfenweise eine wäßrige konzentrierte Natriumcarbonatlösung zugegeben, bis der pH-Wert 11 betrug. Nach 20 h Rühren wurde der Reaktionsansatz mit aktiviertem Ionenaustauscher neutralisiert

und abfiltriert. Die Lösung wurde in vacuo zum Sirup eingeengt und das verbleibende einheitliche Produkt säulenchromatographisch gereinigt.
Ausbeute: 73 bis 77 %

Die Abspaltung der Acyl-Schutzgruppen kann statt mittels Natriumcarbonat auch mit katalytischen Mengen Natriummethylat durchgeführt werden.

Häufig verwendete Laufmittelsysteme zur Chromatographie: Chloroform/Methanol/Wasser 20:5:0,4 und 4:4:1.

AAV 6: Abspaltung der Methylester am Spacer

Zu einer Lösung der Methylester-Verbindung (0,5 mmol) in 30 ml 1,4-Dioxan/Wasser (9:1) wurden unter Rühren mit 1 N Natriumhydroxid-Lösung in Portionen zu 1 ml unter Kontrolle des Laugenverbrauchs (ca. 0,5 mmol NaOH) mit Thymolphthalein als Indikator bei 20°C verseift.
Es wurde mit Ionenaustauscher Dowex 50 WX-8$H^-$ neutralisiert, filtriert, nachgewaschen und in vacuo eingedampft.

Das verbleibende einheitliche Produkt wurde auf einer Säule mit Sephadex G-25 mit dem Eluationsmittel Methanol/Wasser (1:1) gereinigt und lyophilisiert.
Ausbeute: 80 bis 84 %

Laufmittelsysteme für Dünnschichtchromatographie: Chloroform/Methanol/Wasser 5:3:0,5 und 4:4:1.

Verbindung 28 (aus Verbindung 8 nach AAV 4)

N(ß-D-Galactopyranosyl-oxycarbonyl-n-pentylamin)

Verbindung 29 (aus Verbindung 21 nach AAV 2, 3, 5 und 6)

N-((2-Acetamido-2-desoxy-3-O-(ß-D-galactopyranosyl)-

alpha-D-galactopyranosyl)-oxycarbonyl)-L-seryl-5-carboxy-pentylamid

Die Verbindung 29 wurde auch aus Verbindung 22 nach AAV 1, 3, 5 und 4 hergestellt.

Verbindung 30 (aus Verbindung 23 nach AAV 1, 3, 5 und 6)

N-((2-Acetamido-2-desoxy-3-O-(ß-D-galactopyranosyl)-alpha-D-galactopyranosyl)-oxycarbonyl)-L-seryl-L-valyl-5-carboxy-pentylamid

Verbindung 31 (aus Verbindung 24 nach AAV 1, 3, 5 und 6)

N-((2-Acetamido-2-desoxy-3-O-(ß-D-galactopyranosyl)-alpha-D-galactopyranosyl)-oxycarbonyl)-L-seryl-5-carboxy-pentylamid

Verbindung 32 (aus Verbindung 25 nach AAV 1, 3, 5 und 6)

N-((2-Acetamido-2-desoxy-3-O-(ß-D-galactopyranosyl)-alpha-D-galactopyranosyl)-oxycarbonyl)-L-seryl-L-leucyl-5-carboxy-pentylamid

Verbindung 33 (aus Verbindung 26 nach AAV 1, 3, 5 und 6)

N-((2-Acetamido-2-desoxy-3-O-(ß-D-galactopyranosyl)-alpha-D-galactopyranosyl)-oxycarbonyl)-L-seryl-L-isoleucyl-5-carboxy-pentylamid

Verbindung 34 (aus Verbindung 27 nach AAV 1, 3, 5 und 4)

N-((2-Acetamido-2-desoxy-3-O-(ß-D-galactopyranosyl)-alpha-D-galactopyranosyl)-oxycarbonyl)-L-seryl-L-glutamyl(gamma-tert.-butyl)-5-carboxy-pentylamid

Verbindung 35 (aus Verbindung 11 nach AAV 1, 3 und 5)

N-((2-Acetamido-2-desoxy-3-O-(ß-D-galactopyranosyl)-alpha-D-galactopyranosyl)-oxycarbonyl)-L-serin

Verbindung 36 (aus Verbindung 7 nach AAV 1, 3 und 5)

N-((2-Acetamido-2-desoxy-alpha-D-galactopyranosyl)-oxycarbonyl)-aminoethanol

Beispiel 6:

Inhibition von Erdnußlectin (Arachis Hypogea) mit Verbindung 29

Die Hämagglutination von Neuraminidase(VCN)-behandelten Erythrozyten (als 2,5%-ige Suspension) mit Erdunßlectin (Titer $2^{-8}$) wurde vollständig mit Verbindung 29 (0,5 mg/ml) inhibiert.

Beispiel 7:

Bindung der Glycosylurethan-Verbindungen 13, 29, 30, 31, 33, 34 und 35 an aminierte Träger

Die gespacerten Glycopeptidverbindungen, die eine freie kopplungsfähige Carboxygruppe aufweisen, werden entweder direkt mit Carbodiimiden wie z.B. 1-Ethyl-3-(3´-dimethylaminopropyl)-carbodiimid-Hydrochlorid oder als aktivierte Ester wie z.B. N-Hydroxysuccinimid-Derivate an Proteine wie z.B. Rinderserumalbumin, Polypeptide wie z.B. Polylysin oder aminierte Adsorbenzien und Trägermaterialien nach bekannten Verfahren gekoppelt (z.B. in "An introduction to affinity chromatography", C.R. Lowe, NHPC, 1979).

An Rinderserumalbumin, das 35 zugängliche Aminogruppen pro Molekül besitzt, wurden 30 Äquivalente von Verbindung 13 gebunden.

500 Äquivalente von Verbindung 34 wurden an Poly-D-lysin (MG 60.000-80.000) gebunden. Die restlichen freien Aminogruppen des Poly-D-lysins wurden mittels Acetanhydrid acetyliert. Die verbleibende gamma-tert.-Butyl-Schutzgruppe am Glutaminsäure-Baustein der konjugierten Verbindung 34 wurde mit 90%-iger Trifluoressigsäure abgespalten.

Die Verbindung 31 wurde an ein aminiertes Gel (z.B. Kieselgur mit 3-Aminopropylgruppen) in der Konzentration von 10 µmol pro 1 g Träger gebunden.

Patentansprüche:

1. Verbindung der allgemeinen Formel I

$$R^1-(CH_2)_n-CHR^2-CHR^3-NH-CO-O-R^4 \qquad I$$

worin

$R^1$ ein Wasserstoffatom, eine Alkylgruppe mit 1 bis 5 C-Atomen, eine Carboxygruppe, eine Benzyloxycarbonylgruppe oder -CO-Träger

$R^2$ ein Wasserstoffatom, eine Hydroxygruppe, eine tertiäre Alkyloxygruppe mit 4 bis 10 C-Atomen oder $-O-C(CH_3)_2-Ph(OCH_3)_m$ mit m = 0 bis 3,

$R^3$ ein Wasserstoffatom, eine Carboxygruppe, eine $C_1$- bis $C_4$-Alkyloxycarbonyl- oder Benzyloxycarbonylgruppe oder $-CO-A-NH-(CH_2)_a-CO-T$ mit

A ein Bindestrich oder ein in der Natur vorkommender L-Aminosäurerest, bei dem gegebenenfalls die reaktiven Gruppen, die nicht an der Peptidbindung teilnehmen, mit in der Peptidchemie üblichen Schutzgruppen geschützt sind,

T eine Hydroxygruppe, eine O-Alkyl- oder O-Arylalkyl-Schutzgruppe, eine carboxygruppen-aktivierende Gruppe, ein Kephalinrest oder ein Protein, Polypeptid, aminogruppen-tragendes Gel oder Adsorbens und a = 1 bis 10,

$R^4$ ein mit in der Kohlenhydratchemie üblichen Schutzgruppen geschützter oder ungeschützter Glycosylrest, der sich von einer Glycopyranose, Glycofuranose oder einem Oligosaccharid ableitet und n = 0 bis 3 bedeuten.

2. Verbindung der allgemeinen Formel I in Anspruch I

$$R^1-(CH_2)_n-CHR^2-CHR^3-NH-CO-O-R^4 \qquad I$$

worin

$R^1$ ein Wasserstoffamtom, eine Alkylgruppe mit 1 bis 5 C-Atomen, eine Carboxygruppe, eine Benzyloxycarbonylgruppe oder -CO-Träger mit Albumin, Polylysin oder ein aminogruppen-tragendes Gel,

$R^2$ ein Wasserstoffatom, eine Hydroxygruppe oder eine tert.-Butylgruppe oder alpha,alpha-Dimethyl-3,5 dimethoxy-benzylgruppe,

$R^3$ ein Wasserstoffatom, eine Carboxygruppe eine Methyloxycarbonyl- oder Benzyloxycarbonylgruppe oder $-CO-A-NH-(CH_2)_5-CO-T$ mit

A ein Bindestrich oder ein L-Aminosäurerest, bevorzugt Val, Ser, Glu, Glu(t.-But), Leu und Ile und

T eine Hydroxygruppe, eine Methoxy- oder Benzyloxygruppe, eine carboxygruppen-aktivierende Gruppe, bevorzugt O-(N-succinimid)- oder Nitrophenyloxygruppe, ein Kephalinrest oder Trägermolekül, bevorzugt Albumin, Polylysin oder ein aminogruppentragendes Gel,

$R^4$ ein Glycosylrest der Formel II

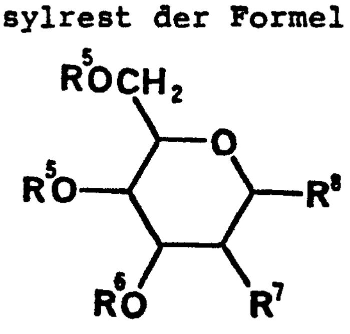

II

in der

$R^5$ ein Wasserstoffatom, eine Acylschutzgruppe, wie Acetyl-, Benzoylgruppe oder eine Benzylgruppe,

$R^6$ ein Wasserstoffatom, eine Acylschutzgruppe, eine Benzylgruppe oder ein ß-D-Galactopyranosyl- oder 2,3,4,6-Tetra-O-acetyl-ß-D-galactopyranosylrest,

$R^7$ ein Wasserstoffatom, eine Hydroxy-, Azido-, Amino-, Acetamido- oder Benzyloxy- oder Trichloracetyloxygruppe und

$R^8$ ein Bindestrich ist, und

n 0 bis 3 bedeutet.

3. Verfahren zur Herstellung einer Verbindung nach Anspruch 1 oder 2, dadurch gekennzeichnet, daß man eine Verbindung der allgemeinen Formel III

$$R^1-(CH_2)_n-CHR^2-CHR^3-NH-CO-O-R^9 \qquad III$$

in der

$R^1$ ein Wasserstoffatom, eine Alkylgruppe mit 1 bis 5 C-Atomen oder eine Benzyloxycarbonylgruppe,

$R^2$ ein Wasserstoffatom oder eine Hydroxygruppe,

$R^3$ ein Wasserstoffatom oder $C_1$ bis $C_4$-Alkyloxycarbonyl- oder Benzyloxycarbonylgruppe,

$R^9$ eine tertiäre Alkyloxygruppe mit 4 bis 10 C-Atomen oder $-O-C(CH_3)_2-Ph(OCH_3)_m$ mit m = 0 bis 3 bedeuten,

mit einer Verbindung der allgemeinen Formel II, in der

$R^5$ eine Acylschutzgruppe oder eine Benzylgruppe,

$R^6$ eine Acylschutzgruppe, eine Benzylgruppe oder ein 2,3,4,6-Tetra-O-acetyl-ß-D-galactopyranosylrest,

$R^7$ ein Wasserstoffatom, eine Hydroxy-, Azido-, Benzyloxy- oder Trichloracetyloxygruppe und

$R^8$ Chlor oder Brom bedeuten,

in einem organischen Lösungsmittel, wie DMF, Dioxan, THF, Essigsäureethylester, Toluol, Chloroform oder Dichlormethan oder deren zwei- oder drei-Komponenten-Mischungen in Gegenwart von Silber-I- oder Quecksilber-II-Salzen, wie Silber-Carbonat-, -perchlorat, -nitrat, -trifluormethansulfonat oder -silikat oder Quecksilbercyanid, -bromid, -chlorid oder -jodid und eines Säurefängers wie Polyvinylpyridin oder Molekularsiebes und gegebenenfalls eines Trockenmittels wie Calciumsulfat oder Magnesiumsulfat bei -50°C bis +80°C, bevorzugt bei -30°C bis +50°C, zu einer Verbindung der allgemeinen Formel I umsetzt.

4. Verwendung der Gruppe

$$-C(CH_3)_2-Ph(OCH_3)_m$$

mit m = 0 bis 3
zum Schutz von alkoholischen Hydroxygruppen.

5. Verwendung einer Verbindung nach Anspruch 1 zur Bindung an einen Träger.

6. Verwendung einer Verbindung nach Anspruch 1 gebunden an einen Träger als Inhibitor, Antigen oder Immunadsorbens.